# EUROPEAN PATENT APPLICATION

(11) **EP 4 509 519 A1**
(43) Date of publication of application: **19.02.2025**
(21) Application number: 24192601.3
(22) Date of filing: 02.08.2024
(51) Int. Cl.: C07K 14/375, A61K 38/16, C12N 1/16

(54) **EXOSOME COMPOSITION CONTAINING GANODERMA IMMUNOMODULATORY PROTEIN AND USES THEREOF**

(30) Priority: 02.08.2023 US 202363517206 P
(71) Applicant: Mycomagic Biotechnology Co., Ltd, New Tapei City 222 (TW)
(72) Inventor: HSEU, Ruey-Shyang, TAIPEI CITY (TW); FU, Hsu-Yuan, TAIPEI CITY (TW)
(74) Representative: Lavoix

(57) **Abstract**

The present disclosure relates to an exosome composition comprising a population of enriched exosomes of fungus containing *Ganoderma* immunomodulatory protein, a recombinant thereof or a fragment thereof, or a mixture derived from the population of enriched exosomes. The present disclosure also relates to a method for treating and/or preventing an oxidative stress related disease in a subject in need thereof, a method for treating and/or preventing inflammation in a subject, and a method for treating and/or preventing a neurological disorder in a subject.

## Description

### PRIORITY INFORMATION

The subject application claims priority to and benefit of U.S. Provisional Patent Application No. 63/517,206, filed August 2, 2023, the content of which is incorporated herein by reference in its entirety.

### SEQUENCE LISTING

The instant application contains a Sequence Listing which is submitted electronically in .xml format and is hereby incorporated by reference in its entirety.

### FIELD OF THE INVENTION

The present disclosure relates to an engineered exosome composition, and uses thereof.

### BACKGROUND OF THE INVENTION

Exosomes are membrane-bound extracellular vesicles (EVs) that are produced in the endosomal compartment of most eukaryotic cells. Exosomes generally have a size range of ~40 nm to 160 nm (average ~100 nm) in diameter with an endosomal origin. Their surface consists of a lipid bilayer from the donor cell's cell membrane, and they contain cytosol from the cell that produced the exosome, and exhibit membrane proteins from the parental cell on the surface. Exosomes were initially thought to be a mechanism for removing unneeded proteins.

There is still a need for expanding the applications of exosomes.

### SUMMARY OF THE INVENTION

The present disclosure provides a novel exosome composition comprising a population of enriched exosomes of engineered fungus, especially yeast.

Accordingly, the present disclosure provides an exosome composition comprising a population of enriched exosomes of fungus containing *Ganoderma* immunomodulatory protein, a recombinant thereof or a fragment thereof, or a mixture derived from the population of enriched exosomes.

In some embodiments, the *Ganoderma* immunomodulatory protein is derived from *Ganoderma lucidum, Ganoderma tsugae, Ganoderma microsporum* or *Ganoderma sinensis.* In some further embodiments, the *Ganoderma* immunomodulatory protein is LZ-8 derived from *Ganoderma lucidum,* FIP-gts derived from *Ganoderma tsugae,* GMI derived from *Ganoderma microsporum,* or FIP-gja derived from *Ganoderma sinensis.*

In some embodiments, the *Ganoderma* immunomodulatory protein comprises an amino acid sequence of SEQ ID NO: 3. In some embodiments, the recombinant of *Ganoderma* immunomodulatory protein comprises an amino acid sequence of SEQ ID NO: 4. In some embodiments, the fragment of *Ganoderma* immunomodulatory protein comprises an amino acid sequence selected from the group consisting of SEQ ID NOs: 1 to 2.

In some embodiments, the fungus belongs to *Saccharomycetaceae* or *Schizosaccharomycetaceae.* Examples of *Saccharomycetaceae* include, but are not limited to, *Kluyveromyces, Cyberlindnera, Debaryomyces, Pichia, Saccharomyces, Kazachstania.* In another aspect, examples of *Schizosaccharomycetaceae* include, but are not limited to *Schizosaccharomyces.* Examples of the yeast include, but are not limited to *Kluyveromyces marxianus (a.k.a. Saccharomyces fragilis, Kluyveromyces fragilis), Kluyveromyces lactis (a.k.a. Saccharomyces lactis), Cyberlindnera jadinii (a.k.a. Candida utilis, Pichia jadinii), Debaryomyces hansenii, Pichia pastoris, Pichia fermentans, Saccharomyces bayanus, Saccharomyces boulardii, Saccharomyces cerevisiae, Saccharomyces florentinus, Kazachstania unispora (a.k.a Saccharomyces unisporus), Kazachstania turicensis (a.k.a. Saccharomyces florentinus),* or *Schizosaccharomyces pombe.* Particularly, in some embodiments of the disclosure, the yeast belongs to *Kluyveromyces lactis (a.k.a. Saccharomyces lactis), Pichia pastoris, Saccharomyces cerevisiae, Hansenula polymorpha, Schizosaccharomyces pombe, Yarrowia lipolytica,* or *Aspergillus niger.*

In some embodiments of the disclosure, the concentration of the enriched exosomes in the exosome composition is greater than about 1 x 10¹⁰ exosomes/mL, about 5 x 10¹⁰ exosomes/mL, about 1 x 10¹¹ exosomes/mL, about 2 x 10¹¹ exosomes/mL, about 4 x 10¹¹ exosomes/mL, about 6 x 10¹¹ exosomes/mL, about 8 x 10¹¹ exosomes/mL, about 1 x 10¹² exosomes/mL, about 2 x 10¹² exosomes/mL, about 3 x 10¹² exosomes/mL, about 4 x 10¹² exosomes/mL, about 5 x 10¹² exosomes/mL, about 6 x 10¹² exosomes/mL, about 7 x 10¹² exosomes/mL, about 8 x 10¹² exosomes/mL, about 9 x 10¹² exosomes/mL, or about 1 x 10¹³ exosomes/mL. In some further embodiments, the concentration of the enriched exosomes in the exosome composition is about 1 x 10¹² exosomes/mL to 2 x 10¹² exosomes/mL.

In some embodiments of the disclosure, the exosome composition comprises a population of enriched exosomes having a mean particle size from about 150 nm to about 250 nm, from about 155 nm to about 245 nm, from about 160 nm to about 240 nm, from about 165 nm to about 235 nm, from about 170 nm to about 230 nm, from about 175 nm to about 225 nm, from about 180 nm to about 220 nm, from about 185 nm to about 215 nm, from about 190 nm to about 210 nm, from about 195 nm to about 205 nm, or about 200 nm.

In some embodiments of the disclosure, the fungus is engineered with a nucleotide encoding the *Ganoderma* immunomodulatory protein, a recombinant thereof or a fragment thereof.

In some embodiments of the disclosure, the fungus is cultivated with a conditioned medium. Examples of the conditioned medium include, but are not limited to, BMGY Medium.

In some embodiments of the disclosure, the exosome composition is obtained by filtration. Examples of the filtration include, but are not limited to, microfiltration, ultrafiltration and nanofiltration. Particularly, the exosome composition is obtained by performing at least one step of tangential flow filtration (TFF). Examples of the TFF include, but are not limited to, performing at least one step of TFF using a TFF membrane with a pore size of about 0.8 µm to about 8 µm , or performing at least one step of TFF using a TFF membrane with a molecular weight cutoff of about 10 kDa to about 100 kDa. In some embodiments of the disclosure, the exosome composition is obtained by performing depth filtration using a membrane with a pore size of about 0.22 µm to about 0.45 µm. In some further embodiments, the exosome composition is obtained by performing TFF using a TFF membrane with a pore size of about 0.8 µm to about 8 µm; performing TFF using a TFF membrane with a molecular weight cutoff of about 10 kDa to about 100 kDa, and performing depth filtration using a membrane with a pore size of about 0.22 µm to about 0.45 µm.

In another aspect, the disclosure provides a method for producing the exosome composition as disclosed herein, comprising cultivating the fungus and obtaining a population of enriched exosomes.

In another aspect, the disclosure provides a method for treating and/or preventing an oxidative stress related disease in a subject in need thereof comprising administrating the exosome composition as disclosed herein to the subject.

In another aspect, the disclosure provides a method for treating and/or preventing inflammation in a subject in need thereof comprising administrating the exosome composition as disclosed herein to the subject.

In another aspect, the disclosure provides a method for treating and/or preventing a neurological disorder in a subject in need thereof comprising administrating the exosome composition as disclosed herein to the subject.

Examples of the neurological disorder include, but are not limited to a neurodegenerative disease, a condition of the nervous system that are secondary to a disease, condition, or therapy having a primary effect outside of the nervous system; an injury to the nervous system caused by trauma; memory loss; or a psychiatric disorder. Particularly, examples of the neurological disorder include, but are not limited to, peripheral nerve damage caused by physical injury, ischemia, prolonged exposure to cold temperature, damage to the central nervous system, dementia, Alzheimer's disease, Huntington's disease, or Parkinson's disease.

The present disclosure is described in detail in the following sections. Other characteristics, purposes and advantages of the present disclosure can be found in the detailed description and claims.

### BRIEF DESCRIPTION OF THE DRAWINGS

FIG. 1 shows that PCR analysis confirms the presence of the gmi gene in the genome of strain mm106. The 339-bp fragments amplified by PCR using GMI-f /GMI-r primers were detected in Lane V and E.
FIG. 2 shows that PCR analysis for yeast species identification. The amplified identical ~300 bp fragments from both mm166 and the host strain.
FIG. 3 shows representative histogram showing particle concentration and size distribution of isolated engineered exosome as measured by nanoparticle tracking analysis (NTA).

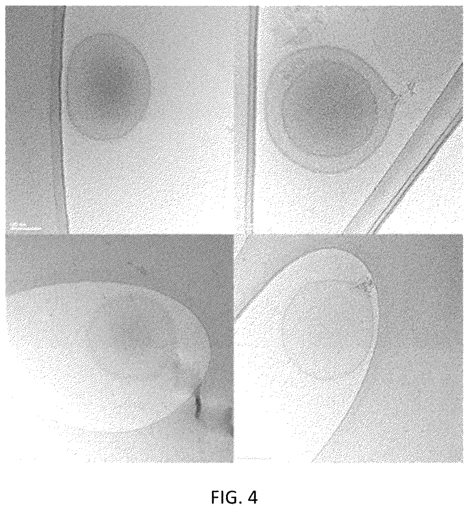

FIG. 4 shows representative cryo-EM image of engineered exosome. Scale bar: 100 nm.

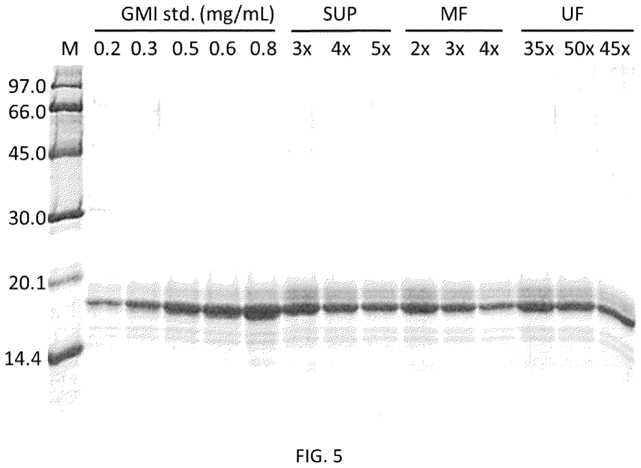

FIG. 5 shows SDS-PAGE analysis of engineered exosomes subjected to different filtration processes, visualized by Coomassie Brilliant Blue (CBR) staining. Lane M: Protein marker with molecular weights indicated in kDa. Lanes 1-5: Standard GMI protein at varying concentrations (0.2, 0.3, 0.5, 0.6, and 0.8 mg/mL) used as protein concentration controls. Lanes 6-8: Supernatant (SUP) from engineered *Pichia pastoris* mm106 fermentate after cell removal via centrifugation, loaded at different dilution folds (3x, 4x, and 5x). Lanes 9-11: Fermentate after microfiltration (MF) for cell clarification, loaded at different dilution folds (2x, 3x, and 4x). Lanes 12-14: Fermentate further processed with ultrafiltration/nanofiltration (UF/NF) for engineered exosome isolation, loaded at different dilution folds (35x, 40x, and 45x).

### DETAILED DESCRIPTION OF THE INVENTION

It is understood that this invention is not limited to the particular materials and methods described herein. It is also to be understood that the terminology used herein is for the purpose of describing particular embodiments and is not intended to limit the scope of the present invention, which will be limited only by the appended claims.

It must be noted that, as used in this specification and the appended claims, the singular forms "a," "an," and "the" include plural referents unless the content clearly dictates otherwise.

As used herein, "about" will be understood by persons of ordinary skill in the art and will vary to some extent with the context in which they are used. If there are uses of the term which are not clear to persons of ordinary skill in the art given the context in which they are used, "about" will mean plus or minus 10% of the particular term.

As used herein, the term "exosome" refers to a small membrane extracellular vesicle of about 30-300 nm diameter that is secreted from producing cells into the extracellular environment. The surface of an exosome comprises a lipid bilayer from the membrane of the donor cell, and the lumen of the exosome is topologically the same as the cytosol from the cell that produces the exosome. The exosome contains proteins, RNAs, lipids, and carbohydrates of the producing cell, though some may be modified or added to the exosome after its release from the cell, either through natural processes or by experimental manipulation.

The term "genetically engineered" or "genetic engineering" of cells refers to manipulating genes using genetic materials for the change of gene copies and/or gene expression level in the cell. The genetic materials can be in the form of DNA or RNA. The genetic materials can be transferred into cells by various means including viral transduction and non-viral transfection. After being genetically engineered, the expression level of certain genes in the cells can be altered permanently or temporarily.

As used herein, the terms "treatment," "treating," and the like, cover any treatment of a disease in a mammal, particularly in a human, and include: (a) preventing the disease from occurring in a subject which may be predisposed to the disease but has not yet been diagnosed as having it; (b) inhibiting the disease, i.e., arresting its development; and (c) relieving the disease, i.e., causing regression of the disease.

The term "preventing" or "prevention" is recognized in the art, and when used in relation to a condition, it includes administering, prior to onset of the condition, an agent to reduce the frequency or severity of or to delay the onset of symptoms of a medical condition in a subject, relative to a subject which does not receive the agent.

As interchangeably used herein, the terms "individual," "subject," "host," and "patient," refer to a mammal, including, but not limited to, murines (rats, mice), non-human primates, humans, canines, felines, ungulates (e.g., equines, bovines, ovines, porcines, caprines), etc. Particularly, the subject is vaccinated.

As used herein, the term "in need of treatment" refers to a judgment made by a caregiver (e.g. physician, nurse, nurse practitioner, or individual in the case of humans; veterinarian in the case of animals, including non-human mammals) that a subject requires or will benefit from treatment. This judgment is made based on a variety of factors that are in the realm of a care giver's expertise, but that includes the knowledge that the subject is ill, or will be ill, as the result of a condition that is treatable by the compounds of the present disclosure.

As used herein, the term "neuron" includes a neuron and a portion or portions thereof (e.g., the neuron cell body, an axon, or a dendrite). The term "neuron" denotes nervous system cells that include a central cell body or soma and two types of extensions or projections: dendrites, by which, in general, the majority of neuronal signals are conveyed to the cell body, and axons, by which, in general, the majority of neuronal signals are conveyed from the cell body to effector cells, such as target neurons or muscle.

As used herein, "neurological disorders" means any physiological dysfunction or death of neurons present in the central nervous system or peripheral nervous system or caused by glia cell dysfunction. A non-limited list of such disorders comprises multiple sclerosis, sciatic nerve defect, brain or code injury, dementia, frontotemporal lobe dementia, Alzheimer's disease, Parkinson's disease, Huntington's disease, prion diseases, neuronopathies and motor neuron disorders. "Neuronopathies" are characterized by neuronal cell death of motor neurons or sensory neurons, and hence neuronopathies can be subdivided into motor and sensory neuron disorders.

The present disclosure provides a novel exosome composition comprising a population of enriched exosomes of *Ganoderma* immunomodulatory protein engineered fungus.

Accordingly, the present disclosure provides an exosome composition comprising a population of enriched exosomes of fungus containing *Ganoderma* immunomodulatory protein, a recombinant thereof or a fragment thereof, or a mixture derived from the population of enriched exosomes.

In one embodiment, the immunomodulatory protein or a recombinant thereof is derived from *Ganoderma lucidum, Ganoderma tsugae, Ganoderma microsporum* or *Ganoderma sinensis.* Preferably, the immunomodulatory protein is LZ-8 derived from *Ganoderma lucidum,* FIP-gts derived from *Ganoderma tsugae,* GMI derived from *Ganoderma microsporum,* or FIP-gja derived from *Ganoderma sinensis* or a recombinant thereof.

In one embodiment, the immunomodulatory protein is derived from *Ganoderma microsporum* (GMI) or a recombinant thereof (reGMI) . More preferably, the immunomodulatory protein (GMI or reGMI) has the amino acid sequence: (1) -Leu-Ala-Trp-Asn-Val-Lys- (LAWNVK; SEQ ID NO: 1) and (2) -Asp-Leu-Gly-Val-Arg-Pro-Ser-Tyr-Ala-Val-(DLGVRPSYAV; SEQ ID NO: 2), the amino acid sequence of: MSDTALIFTLAWNVKQLAFDYTPNWGRGRPSSFIDTVTFPTVLTDKAYTY RVVVSGKDLGVRPSYAVESDGSQKINFLEYNSGYGIADTNTIQVYVIDPD TGNNFIVAQWN (SEQ ID NO: 3) or the amino acid sequence of EAEAEFMSDTALIFTLAWNVKQLAFDYTPNWGRGRPSSFIDTVTFPTVLTDKAYTYRVVVSGKDLGVRPSYAVE SDGSQKINFLEYNSGYGIADTNTIQVYVIDPDTGNNFIVAQWNYLEQKLISEEDLNSAVDHHHHHH (SEQ ID NO: 4).

The choice of host cell is a critical consideration for exosome production, as different cell types have different capacities for exosome production. In some embodiments, the fungus is listed in Table 1.

**Table 1**

| Family | Genus | Species |
|---|---|---|
| *Saccharomycetaceae* | *Kluyveromyces* | *Kluyveromyces marxianus (a.k.a. Saccharomyces fragilis, Kluyveromyces fragilis)* |
| | | ***Kluyveromyces lactis** (a.k.a. Saccharomyces lactis)* |
| *Saccharomycetaceae* | *Cyberlindnera* | *Cyberlindnera jadinii (a.k.a. Candida utilis, Pichia jadinii)* |
| *Saccharomycetaceae* | *Debaryomyces* | *Debaryomyces hansenii* |
| *Saccharomycetaceae* | *Pichia* | ***Pichia pastoris*** |
| | | *Pichia fermentans* |
| *Saccharomycetaceae* | *Saccharomyces* | *Saccharomyces bayanus* |
| | | *Saccharomyces boulardii* |
| | | ***Saccharomyces cerevisiae*** |
| | | *Saccharomyces florentinus* |
| *Saccharomycetaceae* | *Kazachstania* | *Kazachstania unispora (a.k.a Saccharomyces unisporus)* |
| | | *Kazachstania turicensis (a.k.a. Saccharomyces florentinus)* |
| *Saccharomycetaceae* | *Hansenula* | ***Hansenula polymorpha*** |
| *Schizosaccharomycetaceae* | *Schizosaccharomyces* | ***Schizosaccharomyces pombe*** |
| *Dipodascaceae* | *Yarrowia* | ***Yarrowia lipolytica*** |
| *Aspergillaceae* | *Aspergillus* | ***Aspergillus niger*** |

Particularly, in some embodiments of the disclosure, the fungus belongs to *Kluyveromyces lactis* (a.k.a. Saccharomyces lactis), *Pichia pastoris, Saccharomyces cerevisiae,* or *Schizosaccharomyces pombe.*

*P. pastoris* has been extensively used for recombinant protein production since the mid-1980s, with a well-documented safety profile across pharmaceutical, food, and cosmetic industries. Ciofalo et al. (Ciofalo et al., 2006. Safety evaluation of lipase enzyme preparation, expressed in Pichia pastoris, intended for use in the degumming of edible vegetable oil. Regul Toxicol Pharmacol. 45(1):1-8) conducted a comprehensive safety evaluation of a *P*. pastoris-expressed lipase enzyme preparation, further validating the strain's reliability for industrial applications. Additionally, in 1993, the U.S. Food and Drug Administration (FDA) approved the use of *P. pastoris* dried yeast as a food additive in animal feed and drinking water, reinforcing its regulatory safety profile.

In some embodiments, the engineered fungus cells are adapted for exosome production. These eukaryotic species are selected due to their high yield and stability of protein production. The safety of using these eukaryotic species had been well-established. Not willing limited by theory, it is believed that in contrast to mammalian cells, which share the potential for zoonoses when considering the use of exosomes for human therapy, the use of eukaryotic cells offer a potentially safer alternative for exosome production. In some embodiments, appropriate safety measures are taken in the handling and production of these cells to minimize any potential risks.

Host yeast and fungal cell lines are screened for endogenous exosome production using a standard protocol for exosome enrichment and characterization. In some embodiments, briefly, in sub-mL scale, exosomes are isolated using centrifugation and size exclusion filtration, while filtration methods are applied for sub-hundreds liter scale exosome enrichment. Genetic (target specific) engineering of the host cell line is achieved by modified homologous recombination using linear DNA fragments containing homology regions, the desired modification, and a selection marker. In the host cell engineering for exosome production, parameters including transformation strategy/efficiency, genetic engineering target, and selection or screening strategies are all considered.

In some embodiments, the fungus is engineered with a nucleotide encoding the *Ganoderma* immunomodulatory protein, a recombinant thereof or a fragment thereof. The transformation strategy depended on the number of samples handled in a single process and the host cell type. Both physical and chemical methods for the genetic transformation of fungal cells are established. In some embodiments, the transformation comprises a physical method, electroporation, instead of chemical methods, due to its transformation efficiency and condition optimization potential.

The engineering target (cargo load) depends on the desired application of the exosomes. For example, the exosomes are engineered to load bioactive proteins such as antibodies, cytokines, or growth factors - any designed coding sequence. The target proteins are selected based on their function and application.

To select the cells with the highest level of exosome expression, a selection or screening strategy is employed. In some embodiments, antibiotic/auxotrophic selection for cells with stable integration of the genetic material is applied. Antibiotic/auxotrophic selection is used to identify successfully engineered cells. In some embodiments, the engineered host and the corresponding exosomes are specifically characterized for expression of the target molecule(s) using quantitative polymerase chain reaction (qPCR) or Western blotting. The purity, size, and yield of the engineered exosomes are evaluated using NTA. Assays are also used to evaluate the cargo load of the engineered exosomes, such as assessing the protein amount.

Overall, the transfection strategy, genetic engineering target, and selection or screening strategy are optimized based on the specific application of the engineered exosomes. By carefully selecting the target molecule and optimizing the transformation and selection strategies, exosomes with high expression and activity of the desired protein molecule are engineered.

In some embodiments, the culture conditions for the fungal cells are crucial for producing high yields of recombinant proteins and the corresponding exosomes. The culture medium typically include a carbon source, a nitrogen source, trace elements, vitamins, and other components. The choice of these components affected the yield and quality of final products.

Carbon source is an essential component of the culture, with glucose being the most commonly used. Other carbon sources such as maltose, galactose, fructose, and glycerol are also applied.

Cells require nitrogen for the synthesis of amino acids, the building blocks of proteins. In some embodiments, ammonium sulfate is the most commonly used nitrogen source in the culture. Other sources such as yeast extract and peptone are also applied.

In some embodiments, the culture medium contains trace elements. Examples of the trace elements contain, but are not limited to, Iron, zinc, magnesium, and other elements essential for cell growth. In some embodiments, the trace elements are in the form of inorganic salts. In some embodiments, the concentration and ratio of these trace elements, as well as vitamins such as biotin and pantothenic acid, are optimized.

In some embodiments, the culture medium further contains other additives such as buffers, pH adjusters (acid/base), and antibiotics to prevent contamination. In some embodiments, the choice and concentration of these components are optimized.

In some embodiments, other environmental factors including temperature, aeration, and dissolved oxygen level are also optimized. The optimal pH for cell growth and the quality of the final products vary depending on the strain and the genetically engineered target. In some embodiments, the temperature is maintained at a level that promoted cell growth while also allowing for optimal protein folding and stability together with exosome integrity. In some embodiments, aeration is controlled to affect oxygen availability and cell growth rate.

In some embodiments, chemically defined media or animal origin-free (AOF) medium containing well-defined amounts of each component were used to ensure reproducibility and consistency of production quality. A fed-batch culture strategy, where nutrients were added continuously or intermittently, was also adapted for further improvement of the yield.

Not willing limited by theory, it is believed that optimizing culture conditions for fungal cells involve careful control of the medium components, pH, temperature, aeration, and other factors that could affect the yield, quality, and stability of recombinant proteins and exosomes.

In some embodiments, physically-based methods, specifically tangential flow filtration (TFF), are used for exosome isolation to ensure scale-up capability. In some embodiments, engineered exosomes in the engineered cell culture media are isolated using a three-step TFF process followed by nanofiltration. First, cell clarification is performed by subjecting the fermentation broth to microfiltration using a 0.45 µm membrane with 0.22 µm filtered reverse osmosis (RO) water as the dialysis buffer. Further engineered exosome isolation is then achieved through ultrafiltration using a 10 kD membrane with 0.22 µm filtered phosphate-buffered saline (PBS, pH 7.4) as the dialysis buffer. Finally, the isolated engineered exosomes underwent a nanofiltration step using a 0.22 µm membrane.

Not willing limited by theory, it is believed that these physically-based methods offer several advantages over ultracentrifugation for exosome enrichment in both scale-down and scale-up models, especially for subsequent industrial applications. The method are optimized to provide high yields of pure engineered exosomes and were easily scaled up for large-scale production.

In some embodiments of the disclosure, the exosome composition acts as an antioxidant. In another aspect, the disclosure provides a method for treating and/or preventing an oxidative stress related disease in a subject in need thereof comprising administrating the exosome composition as disclosed herein to the subject.

Examples of the oxidative stress related disease include, but are not limited to, Alzheimer's Disease, Parkinson's Disease, Huntington's Disease, amyotrophic lateral sclerosis (ALS), ataxia, Friedreich's ataxia, autism, obsessive-compulsive disorder, attention deficit hyperactivity disorder, migraine, stroke, traumatic brain injury, cancer, inflammation, autoimmune disorders, lupus, MS, inflammatory bowel disease, Crohn's Disease, ulcerative colitis, stenosis, restenosis, atherosclerosis, metabolic syndrome, endothelial dysfunction, vasospasms, diabetes, aging, chronic fatigue, coronary heart disease, cardiac fibrosis, myocardial infarction, hypertension, angina, Prizmetal's angina, ischemia, angioplasty, hypoxia, Keshan disease, glucose-6-phosphate dehydrogenase deficiency, favism, ischemic reperfusion injury, rheumatoid and osteo-arthritis, asthma, chronic obstructive pulmonary disease (e.g. emphysema and bronchitis), allergies, acute respiratory distress syndrome, chronic kidney disease, renal graft, nephritis, ionizing radiation damage, sunburn, dermatitis, melanoma, psoriasis, macular degeneration, retinal degeneration, cataractogenesis, among others.

In another aspect, the disclosure provides a method for treating and/or preventing inflammation in a subject in need thereof comprising administrating the exosome composition as disclosed herein to the subject.

Many other disorders involve oxidative stress and inflammation in affected tissues, including inflammatory bowel disease; inflammatory skin diseases; mucositis related to radiation therapy and chemotherapy; eye diseases such as uveitis, glaucoma, macular degeneration, and various forms of retinopathy; transplant failure and rejection; ischemia-reperfusion injury; chronic pain; degenerative conditions of the bones and joints including osteoarthritis and osteoporosis; asthma and cystic fibrosis; seizure disorders; and neuropsychiatric conditions including schizophrenia, depression, bipolar disorder, post-traumatic stress disorder, attention deficit disorders, autism-spectrum disorders, and eating disorders such as anorexia nervosa.

In another aspect, the disclosure provides a method for treating and/or preventing a neurological disorder in a subject in need thereof comprising administrating the exosome composition as disclosed herein to the subject.

Examples of the neurological disorder include, but are not limited to a neurodegenerative disease, a condition of the nervous system that are secondary to a disease, condition, or therapy having a primary effect outside of the nervous system; an injury to the nervous system caused by trauma; memory loss; or a psychiatric disorder. Particularly, examples of the neurological disorder include, but are not limited to, peripheral nerve damage caused by physical injury, ischemia, prolonged exposure to cold temperature, damage to the central nervous system, dementia, Alzheimer's disease, Huntington's disease, or Parkinson's disease.

The following examples are provided to aid those skilled in the art in practicing the present disclosure.

### Analytical methods used for characterization and quality control

### Nanoparticle Tracking Analysis (NTA)

NTA was performed using a NANOSIGHT^{™} NS300 (MALVERN PANALYTICAL^{®}, UK). Samples were diluted in particle-free phosphate-buffered saline (PBS) to achieve a concentration between 1× 10⁸ and 1x 10⁹ particles/mL. For each sample, five 60-second videos were recorded and analyzed using the NTA 3.0 software. Measurements were performed at room temperature.

### Cryo-Electron Microscopy (cryo-EM)

Exosome samples (1-3 µL) were applied to glow-discharged holey carbon grids and vitrified using VITROBOT^{™} Mark IV (FEI^{®}, USA) at 98% humidity and 4°C. Grids were blotted for 4.5 seconds before being plunged into liquid ethane.

### SDS-PAGE Analysis

Protein samples from different stages of the filtration process were mixed with 4×LAEMMLI^{™} sample buffer (BIO-RAD^{®}) and heated at 95°C for 5 minutes. Samples (20 µg of total protein per lane) were loaded onto a 12% SDS-polyacrylamide gel alongside a protein molecular weight marker. Electrophoresis was performed at 100 V for 90 minutes using a MINI-PROTEAN^{™} TETRA CELL SYSTEM (BIO-RAD^{®}). Gels were stained with COOMASSIE BRILLIANT BLUE R-250^{™} (BIO-RAD^{®}) for 1 hour and de-stained overnight in a solution of 40% methanol and 10% acetic acid. Gel images were captured using IMAGESCANNER^{™} III IMAGING SYSTEM (GE^{®}).

### Protein Quantification

Protein concentrations were determined using the BCA (PIERCE^{™}) according to the manufacturer's instructions, with bovine serum albumin (BSA) as the standard.

### Example 1. Host Cell Selection, Engineering, and Engineered Strain Screening

The engineered strain selected and designated as *Pichia pastoris* strain mm106 was derived using recombinant DNA technology from the commercially available *P. pastoris* KM71H (aox1::ARG, arg4) (INVITROGEN^{®}) strain. This strain was engineered to express the proteins of SEQ ID NOs. 1 to 4, as described in US Patent No. 7,601,808.

### Construction of GMI Expression Vector

The *gmi* gene was cloned from *G. microsporum* using primers with EcoRl/Xbal restriction sites as described in US Patent No. 7,601,808. PCR-amplified DNA fragments were purified by agarose gel electrophoresis, digested with EcoRI and Xbal, and ligated into the corresponding sites of pPICZαA to generate the GMI expression vector.

### Construction and Selection of Strain mm106

Competent *P. pastoris* KM71H cells were prepared according to the Pichia Expression Kit (INVITROGEN^{®}) manual. The GMI expression vector was linearized with Pmel and transformed into competent cells. Potential strains were selected based on antibiotic resistance, with higher resistance correlating with higher recombinant GMI expression levels. The strain designated mm106, which exhibited the highest production level, was selected as the production strain.

### Identity Confirmation of gmi Gene in Production Strain Ey166

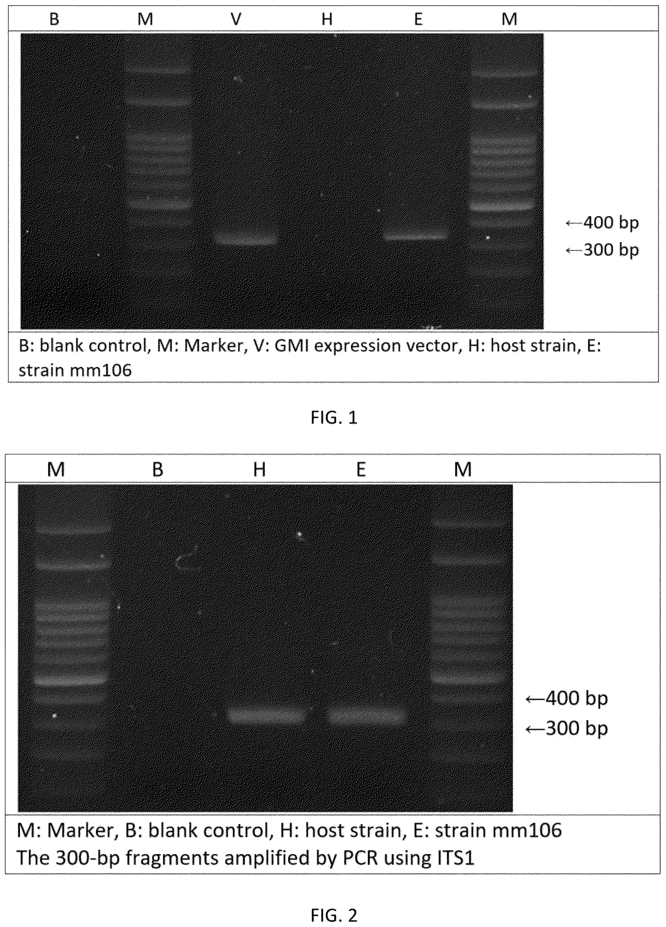

PCR analysis confirmed the presence of the gmi gene in the genome of strain mm106. Primers GMI-f (SEQ ID NO: 5, GAATTCATGTCCGACACTGCCTT) and GMI-r (SEQ ID NO: 6, GTTCCACTGGGCAACAATGAAA), designed from the 5' and 3' ends of the gmi gene, respectively, amplified a 339-bp fragment from mm106 genomic DNA (FIG. 1). This fragment was visualized by agarose gel electrophoresis, while no signal was detected from the host strain.

Furthermore, internal transcribed spacer primers, ITS1 (SEQ ID NO. 7, TCCGTAGGTGAACCTGCGG and ITS4 (SEQ ID NO. 8, TCCTCCGCTTATTGATATGC), used for yeast species identification, amplified identical ~300 bp fragments from both mm166 and the host strain (FIG. 2). Sequencing of these fragments (data not shown) confirmed that both strains belong to the *P. pastoris* species.

### Example 2. Manufacturing Process and Process Controls

### Fermentation:

Frozen cultures (stored in 15% glycerol and at -80°C) were prepared from one batch culture of clone mm106 and inoculated into 400 mL YPD medium to obtain a pre-culture. The pre-culture (O.D.600 >0.4) was inoculated into 3 L YPD medium (initial O.D.600 = 0.05-0.1) to obtain a seed culture. The seed culture (O.D.600 = 12-15) was inoculated into 40 L BMGY medium (initial O.D.600 = 0.6-0.9) for batch fermentation (Agitation: 200-500 rpm; Aeration: air+O₂, 0.5-0.7 vvm; Pressure: 0.1-0.3 kg/cm²; Temperature: 30°C; pH: 5) or fed-batch fermentation (DO spike; Feeding of 5 kg 50% glycerin in 2 hr). The Production fed-batch fermentation was conducted as follows: DO spike; feeding of methanol at the flow rate of 60-80 g/h for 44 h.

### Process After Fermentation:

The cells were subjected to microfiltration of 0.45 µm, 0.6-0.8 m² membrane with dialysis buffer of 0.22 µm filtered RO water. Vesicle isolation was conducted with ultrafiltration by 10 kD, 0.5 m² membrane and dialysis buffer of 0.22 µm filtered phosphate buffered saline, PBS (pH 7.4). The product was subjected to a final filtration with nanofiltration by 0.22 µm, 0.6-0.8 m² membrane.

### Engineered exosome Isolation and characterization

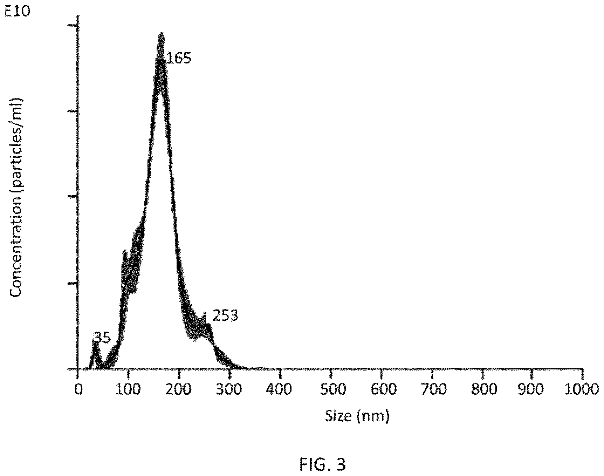

Engineered *Pichia pastoris* mm106 fermentation filtrate was subjected to exosome isolation using tangential flow filtration (TFF). The supernatant was concentrated approximately 10-fold during this process. Nanoparticle tracking analysis (NTA) of the concentrated sample revealed the presence of particles with a diameter ranging from approximately 50 to 250 nm. This size distribution is consistent with that expected for exosomes and small extracellular vesicles (FIG. 3 and Table 2).

**Table 2**

| Stats | | Merged Data | |
|---|---|---|---|
| | Mean | | 162.2 nm |
| | Mode | | 164.2 nm |
| | SD | | 45.0 nm |
| | D10 | | 106.1 nm |
| | D50 | | 161.6 nm |
| | D90 | | 219.7 nm |

| Stats | | Mean +/- Standard Error | |
|---|---|---|---|
| | Mean | | 162.2+/-2.8 nm |
| | Mode | | 163.9 +/- 1.4 nm |
| | SD | | 44.8 +/- 1.1 nm |
| | D10 | | 105.1 +/- 3.4 nm |
| | D50 | | 161.7 +/- 1.5 nm |
| | D90 | | 216.8 +/- 6.2 nm |
| | Concentration (Upgrade) | | 1.48e+12 +/- 4.14e+10 particles/ml |
| | | | 15.5 +/- 0.5 particles/frame |
| | | | 19.7 +/- 0.8 centres/frame |

Cryo-electron microscopy (cryo-EM) was employed to further characterize these particles. Cryo-EM images confirmed the presence of round structures with diameters matching those observed by NTA (50-250 nm). Importantly, these particles exhibited characteristic membrane structures, including both single and double lipid bilayers, providing strong evidence for their identity as extracellular vesicles/exosomes. Within these vesicles, grey regions were observed, indicative of encapsulated proteins, presumed to be the GMI cargo.

To analyze the protein content of the engineered exosomes, SDS-PAGE was performed on samples from different stages of the filtration process. Gels were visualized using Coomassie Brilliant Blue (CBR) staining. The SDS-PAGE results demonstrated that the primary cargo in the engineered exosomes was indeed GMI protein.

Notably, the TFF ultrafiltration process not only achieved a 10-fold concentration of the exosomes but also maintained a high recovery (>85%) of the GMI protein. This high recovery rate underscores the efficiency of the TFF method in concentrating engineered exosomes while preserving their protein cargo.

These findings collectively provide robust evidence for the successful production and isolation of engineered exosomes containing GMI as their primary cargo. The consistency across multiple analytical techniques (NTA, cryo-EM, and SDS-PAGE) strengthens the identification of these particles as exosomes and confirms their intended protein content.

While the present disclosure has been described in conjunction with the specific embodiments set forth, many alternatives thereto and modifications and variations thereof will be apparent to those of ordinary skill in the art. All such alternatives, modifications and variations are regarded as falling within the scope of the present disclosure.

## Claims

1. An exosome composition comprising a population of enriched exosomes of fungus containing *Ganoderma* immunomodulatory protein, a recombinant thereof or a fragment thereof, or a mixture derived from the population of enriched exosomes.

2. The exosome composition of claim 1, wherein the *Ganoderma* immunomodulatory protein is LZ-8 derived from *Ganoderma lucidum,* FIP-gts derived from *Ganoderma tsugae,* GMI derived from *Ganoderma microsporum,* or FIP-gja derived from *Ganoderma sinensis.*

3. The exosome composition of claim 1 or 2, wherein the *Ganoderma* immunomodulatory protein comprises an amino acid sequence of SEQ ID NO: 3.

4. The exosome composition of claim 1 or 2, wherein the recombinant of *Ganoderma* immunomodulatory protein comprises an amino acid sequence of SEQ ID NO: 4.

5. The exosome composition of claim 1 or 2, wherein the fragment of *Ganoderma* immunomodulatory protein comprises an amino acid sequence selected from the group consisting of SEQ ID NOs: 1 to 2.

6. The exosome composition of any one of claims 1 to 5, wherein the fungus belongs to *Kluyveromyces marxianus (a.k.a. Saccharomyces fragilis, Kluyveromyces fragilis), Kluyveromyces lactis (a.k.a. Saccharomyces lactis), Cyberlindnera jadinii (a.k.a. Candida utilis, Pichia jadinii), Debaryomyces hansenii, Pichia pastoris, Pichia fermentans, Saccharomyces bayanus, Saccharomyces boulardii, Saccharomyces cerevisiae, Saccharomyces florentinus, Kazachstania unispora (a.k.a Saccharomyces unisporus), Kazachstania turicensis (a.k.a. Saccharomyces florentinus), Hansenula polymorpha, Schizosaccharomyces pombe, Yarrowia lipolytica,* or *Aspergillus niger.*

7. The exosome composition of any one of claims 1 to 6, wherein the concentration of the enriched exosomes in the exosome composition is greater than about 1 x 10¹⁰ exosomes/mL.

8. The exosome composition of any one of claims 1 to 7, wherein the exosome composition comprises a population of enriched exosomes having a mean particle size from about 150 nm to about 250 nm.

9. The exosome composition of any one of claims 1 to 8, wherein the fungus is engineered with a nucleotide encoding the *Ganoderma* immunomodulatory protein, a recombinant thereof or a fragment thereof.

10. The exosome composition of any one of claims 1 to 9, wherein the exosome composition is obtained by microfiltration, ultrafiltration and nanofiltration.

11. The exosome composition of any one of claims 1 to 10, wherein the exosome composition is obtained by performing at least one step of tangential flow filtration (TFF) using a TFF membrane with a pore size of about 0.8 µm to about 8 µm or with a TFF membrane with a molecular weight cutoff of about 10 kDa to about 100 kDa.

12. The exosome composition of any one of claims 1 to 11, wherein the exosome composition is obtained by performing depth filtration using a membrane with a pore size of about 0.22 µm to about 0.45 µm.

13. The exosome composition of any one of claims 1 to 12, wherein the exosome composition is obtained by performing TFF using a TFF membrane with a pore size of about 0.8 µm to about 8 µm; performing TFF using a TFF membrane with a molecular weight cutoff of about 10 kDa to about 100 kDa, and performing depth filtration using a membrane with a pore size of about 0.22 µm to about 0.45 µm.

14. A method for treating and/or preventing an oxidative stress related disease, inflammation, or a neurological disorder in a subject in need thereof comprising administrating the exosome composition of any one of claims 1 to 13 to the subject.

15. The method of claim 14, wherein the neurological disorder is a neurodegenerative disease, a condition of the nervous system that are secondary to a disease, condition, or therapy having a primary effect outside of the nervous system; an injury to the nervous system caused by trauma; memory loss, a psychiatric disorder, a peripheral nerve damage caused by physical injury, ischemia, prolonged exposure to cold temperature, damage to the central nervous system, dementia, Alzheimer's disease, Huntington's disease, or Parkinson's disease.
